# EUROPEAN PATENT SPECIFICATION

(11) **EP 2 394 168 B1**
(45) Date of publication and mention of the grant of the patent: **05.04.2017**
(21) Application number: 10707661.4
(22) Date of filing: 08.02.2010
(51) Int. Cl.: G01N 33/50

(54) **METHOD AND APPARATUS FOR TESTING TRANSDERMAL MEDICAMENTS**
VERFAHREN UND VORRICHTUNG ZUM TESTEN TRANSDERMALER ARZNEIMITTEL
PROCÉDÉ ET APPAREIL POUR TESTER DES MÉDICAMENTS TRANSDERMIQUES

(30) Priority: 06.02.2009 HU 0900073
(43) Date of publication of application: 14.12.2011
(73) Proprietor: Egis Gyógyszergyár Zrt., 1106 Budapest (HU)
(72) Inventor: MIKULÁSIK, Endre, 8924 Alsónemesapáti (HU); FAZEKAS, Patrik, 9900 Körmend (HU); SZAKÁLY, Péter, 9900 Körmend (HU)
(74) Representative: Stolmár & Partner Patentanwälte PartG mbB
(86) International application number: PCT/HU2010/000017
(87) International publication number: WO 2010/089619

(56) References cited:
- WO-A1-94/02847
- US-A- 4 594 884
- US-A- 4 740 309
- US-A- 4 771 004
- JACOBI U ET AL: "Comparison of four different in vitro systems to study the reservoir capacity of the stratum corneum" 2 March 2005 (2005-03-02), JOURNAL OF CONTROLLED RELEASE, ELSEVIER, AMSTERDAM, NL LNKD- DOI:10.1016/J.JCONREL.2004.11.013, PAGE(S) 61 - 71 , XP004745756 ISSN: 0168-3659 page 62, column 2
- FRANZ T J: "Percutaneous absorption on the relevance of in vitro data" 1 March 1975 (1975-03-01), THE JOURNAL OF INVESTIGATIVE DERMATOLOGY, NATURE PUBLISHING GROUP, GB LNKD- DOI:10.1111/1523-1747.EP12533356, PAGE(S) 190 - 195 , XP003002651 ISSN: 0022-202X figure 1
- NEUBERT R ET AL: "IN-VITRO METHODS FOR THE BIOPHARMACEUTICAL EVALUATION OF TOPICAL FORMULATIONS" 1990, ACTA PHARMACEUTICA TECHNOLOGICA, VOL. 36, NR. 4, PAGE(S) 197-206 , XP009133105 ISSN: 0340-3157 the whole document
- WAGNER H ET AL: "Interrelation of permeation and penetration parameters obtained from in vitro experiments with human skin and skin equivalents" 10 August 2001 (2001-08-10), JOURNAL OF CONTROLLED RELEASE, ELSEVIER, AMSTERDAM, NL LNKD- DOI:10.1016/S0168-3659(01)00396-0, PAGE(S) 283 - 295 , XP004296345 ISSN: 0168-3659 the whole document
- HAWKINS G S ET AL: "Development of an in vitro model for determining the fate of chemicals applied to skin", FUNDAMENTAL AND APPLIED TOXICOLOGY, SOCIETY OF TOXICOLOGY, AKRON, OH, US, vol. 4, no. 2, 1 April 1984 (1984-04-01), pages S133-S144, XP024875978, ISSN: 0272-0590, DOI: 10.1016/0272-0590(84)90145-3 [retrieved on 1984-04-01]
- ADDICKS W J ET AL: "Validation of a flow-through diffusion cell for use in transdermal research.", PHARMACEUTICAL RESEARCH AUG 1987, vol. 4, no. 4, August 1987 (1987-08), pages 337-341, ISSN: 0724-8741

## Description

### Technical field of the invention

The present invention is related to an apparatus for the testing of cosmetic or pharmaceutical formulations intended for transdermal use, a membrane penetration system comprising a penetration cell and a method for testing cosmetic or therapeutical medicinal preparations intended for transdermal use.

### Technical background of the invention

*In vitro* methods suitable for the modelling of biological events including the *in vivo* drug release and absorption-penetration phenomena enjoy distinguished interest in cosmetic as well as pharmaceutical research.

*In vitro* models have great importance during the development of dermal or transdermal formulations used in dermatology, since the absorption and fate of the active ingredient could only be determined in the skin by great difficulties. Quantitative characterization of the therapeutical effect is a complex task as well. General acceptance of the legal and ethical norms related to the forbearance and protection of animals result in sharp decrease in the use of animal models. Testing of the release of the active ingredient however, has great importance in the case of those formulations which are intended to the release of constant daily amounts of the active ingredient dermally.

Tests related to the release of the active ingredient possess distinguished importance in the following areas:
- development of the pharmaceutical dosage form and optimization of the composition thereof,
- targeted monitoring of multicomponent dermal medicaments int he manufacturing phase (e.g. change control, variation of manufacturing technology or the composition of the formulation),
- in-process and release control,
- in bioequivalence studies.

Testing of the penetration of absorbing ointments has increasing role in the formulation and preclinical evaluation of semisolid dosage forms since 1992.

During the therapeutical application of ointments or other dermal medicaments, three processes occur subsequently or simultaneously: (1) release of the active ingredient from the vehicle; (2) penetration of the active ingredient into the outer layers of the skin; (3) permeation following penetration into the living skin, which is sometimes followed by the absorption of the active ingredient into the circulation. The last process is usually not desired when the formulation is intended for topical or dermal use, but may play very important role when the formulation is designed for systemic effect.

In vitro models suitable for the modeling of the release of the active ingredient from transdermal preparations have been classified by several features. NEUBERT and co-workers [Neubert, R., Wohlrab, W.: In Vitro Methods for the Biopharmaceutical Evaluation of Topical Formulations. Acta Pharm, Techn. 36, 197-206 (1990)] distinguished release and absorption models. FARES and ZATZ [Fares, H. M., Zatz, J. L.: Pharm. Technol. 19, (1) 52-58 (1995)] differentiated models based on diffusion or dissolution. The classification of the large number of in vitro models is summarized as follows:
*1. Methods without a membrane*
   1.1. Distribution between liquid having opposite polarity
   1.2. Open tank method
   1.3. Gel diffusion method (agar, gelatine)
*2. Rate-controlled* / *membrane methods*
   2.1. Classification according the apparatus
      2.1.1. Equilibrium diffusion methods in cells
      2.1.2. Flow cell methods
   2.2. Classification according to the type of the membrane
      2.2.1. Membranes of natural origin
         2.2.1.1. Membranes originating from human skin
      2.2.2. Synthetic membranes

The first group of the models is comprised of those methods wherein no membrane is used, while models wherein a natural or synthetic membrane is used between the sample and receptacle compartment, belong to the second group.

Systems without membrane are suitable for the modeling of the release and penetration of the active ingredient.

There are three types of models wherein no membrane is applied: (1) testing the partition between phases having opposite polarity; (2) open-container method and (3) gel model of penetration, wherein the diffusion rate into a gelatine or agar gel is determined.

The latter testing method is based on the assay of the active ingredient diffusing into the recipient. The filter positioned above the test preparation serves as barrier rather than means for the control of diffusion.

Test methods applying a membrane are suitable for modelling all the three phases, i.e. release of the active ingredient, penetration and permeation. In the testing apparatus, the donor and acceptor phase are separated by a membrane. Usually the membrane is of apolar character suitable for modelling the biological barrier. The sample is contained in the donor phase. The acceptor phase is usually distilled water or an aqeuous solution (saline or a buffer optionally containing a surfactant or a transport proteing, e.g. albumine).

Methods wherein a membrane is used are usually classified according to the material of the membrane (e.g. human skin or a part thereof, other biological membranes, or an artificial membrane), the test compound (e.g. isotope-labelled active substance) or by the analytical method. In such a test system, the release and penetration of the active ingredient correspond to the entry into the membrane, while permeation is modelled by the diffusion into the aqeuous acceptor phase.

The release of the active ingredient from the formulation and subsequent biological events can be attributed to the following physical-chemical phenomena: (1) dissolution of the active ingredient in the vehicle; (2) diffusion of the dissolved substances; (3) partition between phases having different polarity.

Dissolution of the active ingredient is dependent primarily on the temperature, the concentration of the active ingredient and the dispersity thereof (particle size, shape, particle size distribution).

The distribution process is primarily controlled by the solubility of the active ingredient. Factors like the quality of the dissolving and distributing media, dissolution-improving auxiliary agents, the temperature and the method of dissolution can play important role in the process.

The diffusion process is primarily controlled by the viscosity of the medium, the gel structure and the physical-chemical structure and properties of the active ingredient and method of preparation thereof.

In the membrane apparatuses according to the state of the art, cells suitable for providing equilibrium diffusion are used. These were replaced later by flow cells, which are suitable for the best simulation of *in vivo* conditions.

*The principle of the modelling of equilibrium diffusion* resides in that the sampling and assay of the active ingredient after predermined periods of time can be used for the determination of the amount of the released active ingredient. Similarly, it is possible to determine the proportion of the active ingredient which has diffused through the membrane. Thus studying the release and penetration of the active ingredient as a function of time become possible.

*The flow cell model* is based on the continous stream of the acceptor phase by a pump. Said acceptor phase is contacted by the membrane and sampled after predetermined periods of time or the concentration of the active ingredient is monitored and assayed continously online (e.g. using a spectrophotometer equipped with flow cell).

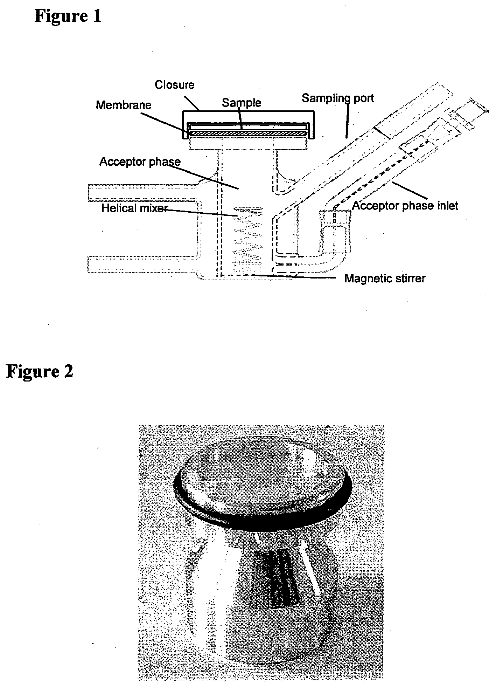

Among the in vitro models, the Microette ^{™} transdermal diffusion cell designed and manufactured by Hanson is considered the most reproducible and in general, the most suitable for the modelling *in vivo* conditions. In this apparatus, the compartment containing the test sample (donor compartment) is separated by a special membrane from the acceptor phase. The position of the membrane is either vertical or horizontal and the volume and surface area of the stirred cell can be selected from several variants. It is possible to use several cells at the same time. Sampling and flow control of the acceptor phase is automated and simultaneous in each cell. The temperature of the cells can be kept constant due to the dependence of solubility and diffusion constant on temperature. Similarly, the flow of the acceptor phase is thermostated. Such cells are known from the art as Franz cells [Franz, T.: Curr. Probl. Dermatol. 7, 58-68 (1978)]. The structure of the known cell is shown in Figure 1.

The most important part of the equipment is the penetration cell, wherein the test ointment, gel, cream etc. is present in a closed compartment separated from the acceptor phase by a membrane. The assay of the active ingredient which passed the membrane is usually carried out by high-performance liquid chromatographic analysis in the acceptor phase.

In US 4,740,309 is described an apparatus for determining the rate of movement of a study substance through a membrane. The study substance is applied to the external side of a membrane, i.e. to a cell cap side of the membrane opposite to a passage through which is flown a receptor fluid. The apparatus comprises a closed sample compartment, i.e. a compartment in which environmental factors are given and constant. In fig. 8 is disclosed a conventional diffusion cell which comprises chambers located on opposite sides of a membrane. One chamber forms a donor chamber and is adapted to receive a dose of a chemical substance under study. The study substance is absorbed through the membrane and enters a receptor cell situated on the opposite side of the membrane. The receptor cell contains a receptor or bathing fluid into which the study substance enters. The receptor fluid is maintained at a constant temperature by thermostatically controlled fluid which circulates through a jacket surrounding the receptor chamber. The donor chamber is formed by a cell cap which is suitably clamped to the cell body.

U. Jacobi et al. Journal of controlled release 103 (2005) 61 - 71 (D2) describe a study on the reservoir capacity of porcine stratum corneum (SC) for flufenamic acid and its drainage via penetration into deeper skin layers. Four different in vitro systems were used in this study: Franz diffusion cell using full thickness skin and split skin, Saarbrücken penetration model and intact porcine tissue.

G. Hawkins et al. Fundamental and Applied Toxicology 4, S. 133-S144 (1984) describe an *in vitro* model for determining the fate of chemicals applied to skin. The percutaneous penetration and evaporation of N,N-diethyl-m-toluamide were determined on skin samples immediately after excision and after a freezing period of 1 to 6 weeks at -80 °C. Variation of flow rate and type of acceptor fluid did not significantly influence percutaneous penetration whereas air temperature had a large influence on penetration and evaporation of the test compound. The apparatus used for performing the tests is not described in detail.

### Summary of the invention

The present invention is based on the recognition that applying the methods and using the apparatus known from the state of the art, the modelling of the behaviour of preparations applied to the skin is not possible. It has been perceived that the environmental factors influence the behaviour of preparations applied to the skin. Thus, the effect of the environmental variables can be modelled by a membrane model wherein the compartment containing the test preparation is open to the environment. Although there is disclosure in the art related to open, equilibrium diffusion apparatus and different kinds of membrane diffusion cells, the state of the art is silent about a penetration apparatus comprising a membrane diffusion cell having open sample compartment. The prior art also lacks disclosure regarding the use of an open sample compartment apparatus in conjuction with controllable environmental conditions.

Furthermore, it has been recognized that methods according to the state of the art do not consider the fact that the properties and composition of the test preparation are not constant during the testing period but they are changing as a function of several environmental factors. Such factors are the quality of the skin area where the test preparation is used (.e.g. face or hands are usually exposed to light and air flow; the abdomen or groin are usually covered by clothing and thus isolated from air flow and light); the flow rate and humidity of the air flow in the vicinity of the exposed skin and the temperature difference of the skin compared to the surrounding air; intensity, duration and wavelength of the light exposition of the skin.

Thus it becomes possible to consider and model the effect of environmental conditions according to the body surface (e.g. face generally exposed to air flow and light; abdomen or groin isolated from air and light) where the use of the test preparation is intended.

In many cases, it is necessary to study these relationships in detail, e.g. during the development of the intended therapeutical application (whether a light-protecting, antiinflammatory cream is suitable for its function under extreme environmental or weather conditions, such as during skiing in cold, windy winter weather or during sunbathing in hot, stagnating air in the tropical summer) and in the development of formulation intended for special use (development of climate- withstanding formulations)

### Detailed description of the invention

The objective of our research-development work was to develop a new testing apparatus and method, which is allows the testing of pharmaceutical formulations applied to the skin under variable environmental conditions using an open compartment membrane Penetration method.

According to the invention, an apparatus for the testing of cosmetic or pharmaceutical formulations intended for transdermal use has been constructed, which comprises an acceptor phase conditioning and delivery system and a membrane penetration system having a penetration cell, characterized in that the penetration cell is an open sample compartment membrane penetration cell comprising a cell body made of an inert material with regard to an acceptor phase and a test preparation, wherein in the cell body is located a chamber for the acceptor phase and is provided with acceptor phase inlet and outlet, and a membrane located on the upper part of the cell body and held in place by means suitable for fastening, wherein the membrane is for locating the test formulation on an upper surface of the membrane and wherein a compartment containing the test preparation is open to an environment, wherein the cell body has a weight and a heat capacity such that the fluid stream is brought to the temperature of the cell already at the inlet and the membrane penetration system does not comprise a separate temperature control for the acceptor phase and the apparatus further comprises a sampling and an analytical system and a control system for controlling one or more environmental factors at the membrane surface of the open sample compartment of the membrane penetration cell.

The apparatus according to the present invention is suitable for the prediction of the *in vivo* properties of a transdermal pharmaceutical formulation. It is well suited for the approximation of the physiological conditions of the human skin and the transdermal pharmaceutical compositions can be tested under environmental conditions similar to those of the real-time application. Using the apparatus of the present invention, it becomes possible to study the surface effects during the application of transdermal preparation. According to the method of the present invention, testing and *in vitro* modeling of any transdermal preparation, including but not limited to compositions having high concentration of volatile components, e.g. gels, emulsions, Solutions for external use, suspensions, powders, aerosols etc.

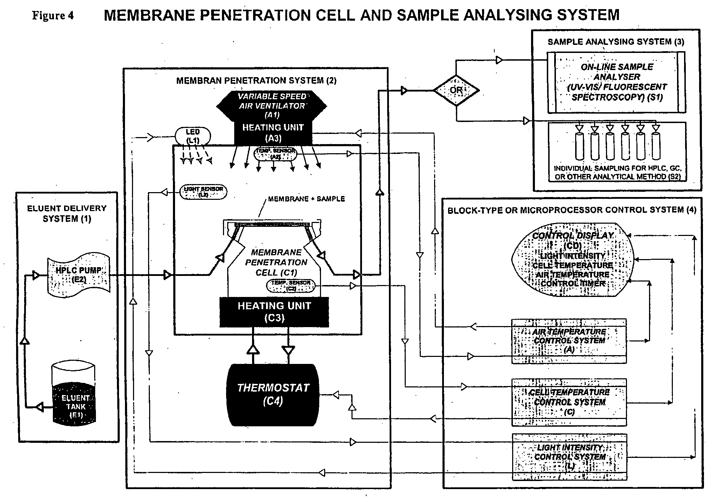

The Systems and their connections of the apparatus according to the present invention are depicted in Figure 4. The membrane penetration system consists of a membrane penetration cell having open sample compartment (C1), system for controlling environmental factors, delivery system for the acceptor phase stream (1), sampling and analytical system (3). The apparatus is controlled by a block-type or microprocessor-type controller (4).

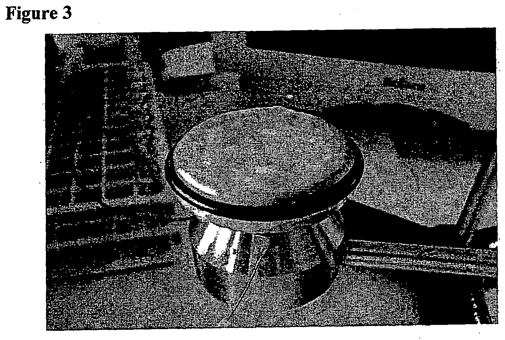

According to a further aspect of the present invention, there is provided a membrane penetration cell system comprising a penetration cell having an open sample compartment wherein the penetration cell comprises a cell body made of an inert material with regard to an acceptor phase and a test preparation, wherein in the cell body is located a chamber for the acceptor phase and the cell body is provided with acceptor phase inlet and outlet, and a membrane located on the upper part of the cell body and is held in place by means suitable for fastening, wherein the membrane is for locating the test formulation on an upper surface of the membrane, wherein the compartment containing a test preparation is open to an environment, wherein the cell body has a weight and a heat capacity such that the fluid stream is brought to the temperature of the cell already at the inlet and the membrane penetration system does not comprise a separate temperature control for the acceptor phase, a temperature control system and means suitable for adjustment and control of one or more environmental factors at the surface of the open sample compartment of the membrane penetration cell. The cell can be made of any suitable inert material known from the state of the art, considering the properties of the acceptor phase and the test preparation, e.g. steel. The membrane is located on the upper part of the cell body. The membrane is held in place by means suitable for fastening, e.g. a threaded fastening ring or an elastic fastening ring. The cell body, wherein a chamber for the acceptor phase is located, is provided with acceptor phase inlet and outlet and optionally a jacket is provided suitable for attaching a liquid thermostat for temperature adjustment and control (Figure 2, Figure 3). Optionally, thermal insulation is provided at the outer side of the cell body. The cross-sectional area of the cell body at the membrane side is precisely determined by measurement and/or calibration. One possible embodiment of the cell is shown in Figure 2. The same cell loaded with a cream sample is shown in Figure 3.

In one embodiment of the apparatus according to the present invention depicted in the flow chart of Figure 4, the membrane penetration cell having open sample compartment is located in a cabinet provided with glass doors at each side. Parts of the cell are the cell body (C1) with the thereto attached membrane and elastic fastening ring and the fluid connections suitable as inlet and outlet of the acceptor phase. The cell body is made of AISI 36 grade stainless steel block with the provision of borings for the sensors. The cell body can be removed from the apparatus for cleaning and sanitization. The weight of the cell is approximately 500 g, which is significantly greater than the weight of conventional cells known from the prior art. Due to the relatively great weight, heat capacity and compact design of the cell, no separate temperature control for the acceptor phase is required since the fluid stream is brought to the temperature of the cell already at the inlet. The cross-sectional area and the volume of the cell is precisely determined (10.00 cm² and 3,00 cm³, respectively) thereby facilitating the calculation of the results.

The temperature of the cell is controlled by a convective radiator (C3) made of nickel-plated brass, which is heated/cooled by a heat transfer medium delivered by a liquid thermostat (C4). The heat transfer medium is an antifreeze. The thermostat is electrically heated and if desired, cooling of the heat transfer medium is provided by an external cooling unit. The temperature can be set and controlled with 0.1 °C precision. The temperature sensor (C2) is located in the cell interior. The signal proportional to the cell temperature is delivered to a control circuit (Figure 4, C). The control rate of the system is very fast (time constant is less than 30 seconds). The heating is controlled by a variable width square wave signal. The heating element is switched by a solid state relay. When cooling is necessary, the precooled heat transfer medium is heated as much as necessary to maintain the preset temperature.

In the apparatus, a LABOR-MIM ultratermostat (C4, Labor MIM, Esztergom, Hungary) is used with 2 liters of glycerol-distilled water (50:50, v/v) heat transfer medium. The temperature sensor (C2) is a NIVELCO CTFP-121-1 (Nivelco, Budapest, Hungary) Pt/100/B platinum heat sensor, the temperature control (Figure 4, C) is provided by a block-type OMRON E5CSV-R1 T-500 (Japan) temperature controller.

Any type of membrane suitable for test purposes can be used in the apparatus according to the present invention. It is possible to use natural membranes (e.g. of human origin or obtained from mammals, e.g. swine, rat, rabbit skin or layers thereof, membrane obtained by means of cell propagation etc.) or artifical membranes made of biologically or synthetically obtained materials, e.g. polysiloxane (Silastic), cellulose or derivatives thereof, vinylacetate or acrylate polymers. The apparatus according to the present invention is suitable for the use with skin membrans or membranes comprised of layers of skin (e.g. epidermis, upper dermis, stratum corneum etc.) prepared by dermatomization, heat treatment, chemical or enzymatic treatment etc. Preparation of membranes suitable for the absorption-penetration studies is carried out by using methods known from the state of the art.

The sample of the test formulation is located on the upper surface of the membrane used for modelling of skin or other biological barriers. The amount of the sample is approximately 0.001-0.1 g/cm² calculated for the area of the membrane.

The sample can be exposed according to the present invention to an air flow having controlled flow rate, temperature and if desired, controlled humidity. The apparatus according to the present invention is suitable to provide an air stream with controlled flow rate at a predetermined temperature and humidity by an air delivery system (compressed air cylinder or central compressor, ventillator, HVAC equipment etc), optionally air cleaning means, temperature controlling system and humidity controlling system. It is also possible to measure and record the heat content and humidity of the air leaving the apparatus according to method known from the art..

According to one embodiment of the present invention, the air flow is provided by a voltage-controlled, variable rpm, low voltage ventillator (A1). The temperature of the air stream is controlled by means of an electric heating unit, air temperature sensor (A2) and control system (Figure 4, A). Cooling is possible by feeding the ventillator with pre-cooled air stream and heating thereof by the above-described method. Fast control rate is possible by the variable
Cooling is possible by feeding the ventillator with pre-cooled air stream and heating thereof by the above-described method. Fast control rate of air temperature is possible by the variable pulse-width square wave controlled, low voltage direct heating heating element (time constant less than 30 sec).

Air flow rate is controlled by voltage control of the ventillator between 0.5 and 2.5 m/s. Air flow is calibrated as the function of control voltage over the membrane surface.

The temperature of the heating element (A3) is controlled by the signal of the temperature sensor (A2, NIVELCO CTFP-121-1 Pt/100B platinum heat sensor). The controller (C) is an OMRON E5CSV-R1 T-500 unit. The air flow is provided by an INC A199411 (R= 40mm) ventillator controlled by an LM7815 voltage controller. The voltage of the ventillator can be continously adjusted between 3 and 15 volts. In one embodiment, the relative humidity of the air entering the apparatus is identical to that of the ambient air. However, the air humidity can be controlled using any method known from the prior art.

The test sample applied to the membrane surface can be exposed to light. This facilitates the observation of physical-chemical changes occuring in the sample as well. According to one method, lighting is provided by a light-emitting diode (L1) having similar emission spectrum to sunlight (YOLDAL YZ-WS5S20, Sunny-White, max light intensity 11.000 mcd). The light exposure is measure by a sensor (L2) and the emission of L1 is controlled accordingly. Light intensity can be adjusted by frequency control of the excitation voltage of L1 using an in-house built controller. In this way, the emission spectrum of the LED does not depend on light intensity.

In those cases when the test preparation is subjected to light having different intensity or emission spectrum from that of the normal sunlight (e.g. when the test preparation is applied at high altitudes above the sea level where the intensity of ultraviolet radiation is significantly higher than at sea level), it is possible to substitute the light source with a different one having similar emission spectrum and intensity to that expected under the circumstances of the real-life use.

If desired, alterations of the test preparation resulting from the exposure to environmental factors can be recorded using an imaging method for subsequent evaluation simultaneously with or alternatively to visual inspection.

Penetration measurement is carried out by providing a stream of the acceptor phase with constant flow rate, composition and temperature, causing said stream of the acceptor phase flowing through the penetration cell and determining the concentration of the active or characteristic ingredient of the test preparation in the effluent stream leaving the membrane penetration cell (1) having open sample compartment as a function of acceptor phase volume or time (Figure 4).

Concentration of the active ingredient or that of a characteristic component of the test preparation can be carried out on-line by assaying the analyte concentration with an on-line analytical system (3), e.g. spectrophotometry using an apparatus equipped with a flow-through cuvette. Altervatively, the assay of said analytes can be carried out by sampling the effluent acceptor phase stream at predetermined periods of time or effluent volume and determining the concentration of the analyte using any analytical method (Figure 4). Preferably, a liquid-phase assay is used. It is also preferable to use an assay method providing seelective assay of the analyte. Chromatographic methods can be preferably used.

During the sampling process for the purpose of on-line assay, it is important to keep the outlet of the cell at the same height to that of the membrane to prevent pressure buildup at the membrane.

The delivery of the acceptor phase is preferably carried out by pumping said liquid. For this purpose, any pump can be used which is suitable for the delivery of the acceptor phase at the required flow rate with the desired precision. The flow rate of the acceptor phase during the penetration testing experiments falls within the magnitude of the flow rate of body fluid, e.g. blood flow. However, the flow rate of the acceptor phase can be influenced by the pressure resistance of the membrane used during the testing. During the experiments, the flow rate of the acceptor phase is usually between 0.5 and 3 ml/min, preferably 0.1 to 0.5 ml/min. The fluctuation of the flow rate must not exceed 1 percent relative to the actual flow rate. Preferably, a volume displacement pump can be used. The inlet and outlet ports of the cell may be provided with suitable threads in order to allow the use of low dead volume tubing and connectors developed for high-performance liquid chromatographic systems.

For example, components of a liquid chromatography system can be used for the delivery of the acceptor phase. An eluent tank can be used as a reservoir for the acceptor phase (E1). Preferably, a heat and chemically resistant glass bottle, e.g. a TORAX glass bottle can be used. The acceptor phase is delivered to the penetration cell by the high-pressure pump of the liquid chromatographic system. Pumps (E2) used in high-performance liquid chromatographic systems can be advantageously used due to their high precision and low flow fluctuation (Figure 4).

It is especially important to prevent any pressure buildup in the cell, since pressure difference disturb the membrane diffusion process. In this order, the cross sectional area of the inlet tubing is kept smaller than that of the outlet tubing, preferably the cross sectional area of the inlet tube is ¼ of the outlet tube.

The acceptor phase is usually a solution suitable for modelling physiological liquids. Suitable acceptor phases are purified water, saline, phosphate-buffered saline or a Ringer-solution.

The acceptor phase is degassed prior to use according to one of the methods known from the art, i. e. sonication or gas purging. Preferably, argon purge is used. If available, the on-line degassing module of a high-performance liquid chromatographic appararatus can be used.

The inventors of the present application used as eluent delivery module of a Shimadzu LC-6A Liquid Chromatograph or Agilent (Hewlett Packard) HP G 1311A high-pressure pump attached to an ERMA ERC-3312 or Agilent (Hewlett Packard) G 1322 A degassing unit are suitable for acceptor phase delivery.

Detectors (S1) having flow cells built for the purpose of liquid chromatography can also be used as means for on-line assay of the analyte (Figure 4). The inventors of the present application used an Agilent (Hewlett Packard) G1315A photodiode array detector or a Shimadzu UV-Spectrophotometric Detector as well as a Beckman Fluorescent Detector has been successfully used for this purpose. Detection parameters, such as detection wavelength or excitation/emission wavelength can be selected according to the properties of the analyte. In the case there are no means for the specific detection of the analyte, a mass-sensitive detector (e.g. refractive index or evaporative light scattering detector) can be used. The detector signals are acquired and evaluated using a computerized data acquisition system.

The control of the apparatus optionally can be carried out by a computerized process control system including the measurement and control of critical parameters, data acquisition and analysis rather than using individual electronic or electromechanic control systems.

It is also possible to build a multitude of cells and sampling/analytical system into one apparatus thus facilitating simultaneous testing.

According to a further aspect of the present invention, there is provided a method for *in vitro* modelling the release of the active ingredient from a pharmaceutical or cosmetic test preparation, especially the modelling of the absorption-penetration processes.

In the method according to the invention for testing cosmetic or therapeutical medicinal preparations intended for transdermal use, a sample is transferred to the membrane attached to the surface of the penetration cell described above, and said sample is exposed to preselected and controlled environmental factors with simultaneously delivering a controlled flow of constant temperature acceptor phase through the cell body and the concentration of the pharmaceutically active ingredient or a characteristic constituent of the test sample in the effluent is determined.

The method carried out as following. The acceptor phase is filtered and degassed, the cell is degassed and the membrane is attached thereto. After stabilization of flow rate, temperature, air flow and light intensity (usually 1 to 24 hours), the test preparation is transferred to the membrane homogeneously in 1 to 3 seconds. The amount of the test formulation is preferably an amount applied under real-time conditions, or practically one dose unit of the preparation under testing.

Thereafter the flow of the acceptor phase is resumed and in case of on-line analysis, the concentration of the active ingredient of the preparation or that of a characteristic component of the same is recorded. The calculation of the actual concentration can be carried out by external calibration as a function of acceptor phase volume or the elapsed time. From these data, the rate of dissolution is determined. Alternatively, in the case of off-line analysis, fractions are collected and the analyte is assayed in each fraction by a suitable analytical method known from the art. From these data, the amount of the analyte present in the corresponding fraction is determined and the rate of dissolution is calculated.

### Example 1

### Membrane penetration testing of Troxerutin 5% gel

The test preparation contains 5 % troxerutin as active ingredient. In the test, 0.9 weight% sodium chloride solution is used as an acceptor phase. The flow rate of the acceptor phase is 1 ml/min. The membrane used in the testing was cellophane, 10 cm width by 10 cm length. The temperature of the cell is 34 °C. The cell is exposed to natural daytime light. In the first (I) test, no artificial air flow is applied. In the second test set (II), the linear air flow rate is 2 m/sec. The concentration of the active ingredient is determined by ultraviolet spectrometry on-line at the wavelength of 349 nm.

The membrane together with the apparatus is allowed to stabilize for 60 minutes. Thereafter, an amount of approx. 300 mg of the test preparation measured by analytical precision is transferred homogeneously onto the membrane in 2 to 3 seconds. Thereafter the flow of the acceptor phase is resumed and the concentration of the dissolved active ingredient is recorded in the acceptor phase.

The amount of the active ingredient dissolved in test set (I) and (II) are shown in Table 1 (relative to the amount of the active ingredient applied to the membrane).

**Table 1. Membrane penetration testing of Troxerutin 5% gel**

| **Time (min)** | **Released amount (%) Test set (I)** | **Released amount (%) Test set (II)** |
|---|---|---|
| 0 | 0 | 0 |
| 2 | 1.02 | 1.17 |
| | 2.52 | 3.03 |
| 6 | 4.84 | 5.54 |
| 8 | 5.29 | 6.68 |
| 10 | 5.29 | 7.06 |
| 20 | 5.04 | 7.31 |
| 30 | 4.54 | 7.09 |
| 40 | 4.03 | 6.55 |
| 50 | 3.68 | 5.80 |
| 60 | 3.24 | 4.59 |
| 120 | 2.51 | 2.02 |
| 180 | 1.68 | 1.02 |
| 240 | 0.85 | 0.32 |
| 300 | 0.47 | 0.11 |
| 360 | 0.26 | 0.02 |

### Example 2

### Comparative membrane penetration testing of a piroxicam-containing cream using cellophane and human skin membranes

The test preparation contains 1% piroxicam as active ingredient. In the test, the acceptor phase comprises 0.9 weight% sodium chloride solution. The flow rate of the acceptor phase is 0.3 ml/min. During the first test series (I), a cellophane membrane, during the second test series (II), a human skin membrane was used. Temperature of the cell was kept at 34 °C. During both test series, the cell was exposed to natural sunlight. No air flow was applied.

The membrane was allowed to stabilize for 60 minutes.

After the stabilization period, 300 to 400 mg of test preparation measured by analytical precision are applied to the membrane homogeneously in 2-3 seconds. The flow of the acceptor phase is resumed and effluent fractions for periods of 30 minute each are collected. The piroxicam and nipagin M concentration of each fraction is determined by high-performance liquid chromatography. Using the volume and concentration of each analyte, the amounts of piroxicam and Nipagin M are calculated for each fraction.

The high-performance liquid chromatographic analysis is carried out using external standard method in a reversed phase system. The separation is carried out using a Nucleosil C18 (250 mm x 4,6 mm i.d.) column and a buffer-acetonitrile-methanol (1000:660:340, v/v/v) mobile phase. The separation is performed at the temperature of 40 °C. Flow rate of the mobile phase is 1.0 ml/min. Detection is carried out by ultraviolet photodiode array detector in the wavelength range of 250 to 450 nm. The relative amount of the released piroxicam and Nipagin M (methyl-4-hydroxybenzoate) to the amount applied to the membrane in test series (I) and (II) is given in Table 2.

**Table 2 Cumulative relative amount of the dissolved piroxicam and Nipagin M related to the amount applied to the membrane in test series (I) and (II), respectively**

| **Fraction No.** | **Time (min)** | **Cumulative amount of dissolved piroxicam, % Series (I)** | **Cumulative amount of dissolved piroxicam, % Series (II)** | **Cumulative amount of dissolved Nipagin M, % Series (I)** | **Cumulative amount of dissolved Nipagin M, % Series (I)** |
|---|---|---|---|---|---|
| 1. | 0-30 | 0.23 | 0.00 | 63.01 | 1.77 |
| 2. | 30-60 | 0.47 | 0.00 | 88.79 | 7.56 |
| 3. | 60-90 | 0.68 | 0.00 | 100.7 | 17.16 |
| 4. | 90-120 | 0.89 | 0.00 | | 29.32 |
| 5. | 120-150 | 1.09 | 0.01 | | 41.74 |
| 6. | 150-180 | 1.30 | 0.02 | | 53.72 |
| 7. | 180-210 | 1.50 | 0.02 | | 64.31 |
| 8. | 210-240 | 1.69 | 0.03 | | 73.57 |
| 9. | 240-270 | 1.88 | 0.04 | | 81.34 |
| 10. | 270-300 | 2.08 | 0.05 | | 87.95 |
| 11. | 300-330 | | 0.05 | | 94.19 |
| 12. | 330-360 | | 0.06 | | 100.0 |

## Claims

1. Apparatus for the testing of cosmetic or pharmaceutical formulations intended for transdermal use, which comprises an acceptor phase conditioning and delivery system (1) and a membrane penetration system (2) having a penetration cell (C1), **characterized in that** the penetration cell (C1) is an open sample compartment membrane penetration cell comprising a cell body made of an inert material with regard to an acceptor phase and a test preparation, wherein in the cell body is located a chamber for the acceptor phase and is provided with acceptor phase inlet and outlet, and a membrane located on the upper part of the cell body and held in place by means suitable for fastening, wherein the membrane is for locating the test formulation on an upper surface of the membrane and wherein a compartment containing the test preparation is open to an environment, wherein the cell body has a weight and a heat capacity such that the fluid stream is brought to the temperature of the cell already at the inlet and the membrane penetration system does not comprise a separate temperature control for the acceptor phase and the apparatus further comprises a sampling and an analytical system (3) and a control system (4) for controlling one or more environmental factors at the membrane surface of the open sample compartment of the membrane penetration cell.

2. Apparatus according to claim 1, wherein the inert material with regard to an acceptor phase and a test preparation is stainless steel.

3. Membrane penetration system (2) comprising a penetration cell (C1) having open sample compartment wherein the penetration cell (C1) comprises a cell body made of an inert material with regard to an acceptor phase and a test preparation, wherein in the cell body is located a chamber for the acceptor phase and the cell body is provided with acceptor phase inlet and outlet, and a membrane located on the upper part of the cell body and is held in place by means suitable for fastening, wherein the membrane is for locating the test formulation on an upper surface of the membrane, wherein the compartment containing a test preparation is open to an environment, wherein the cell body has a weight and a heat capacity such that the fluid stream is brought to the temperature of the cell already at the inlet and the membrane penetration system does not comprise a separate temperature control for the acceptor phase, a temperature control system (C2, C3, C4) and means (L1, L2, A1, A2, A3) suitable for adjustment and control of one or more environmental factors at the surface of the open sample compartment of the membrane penetration cell.

4. Membrane penetration system (2) according to claim 3, **characterized in that** the adjustable and controllable environmental variables at the open sample compartment of the membrane penetration cell are selected from light intensity, light wavelength, air flow rate, air temperature and air humidity.

5. Membrane penetration system (2) according to claim 3 or 4, **characterized in that** the penetration cell (C1) having open sample compartment comprises a thermal insulation provided at the outer side of the cell body.

6. Membrane penetration system (2) according to one of claims 3 to 5, wherein the inert material with regard to an acceptor phase and a test preparation is stainless steel.

7. Method for testing cosmetic or therapeutical medicinal preparations intended for transdermal use, which comprises transferring a sample to the membrane attached to the surface of the penetration cell according to claim 3, exposing said sample to preselected and controlled environmental factors with simultaneously delivering a controlled flow of constant temperature acceptor phase through the cell body and determining the concentration of the pharmaceutically active ingredient or a characteristic constituent of the test sample in the effluent.

8. Method according to claim 7, **characterized by** that one or more environmental factors selected from temperature, light intensity, light wavelength, air flow rate, air temperature, air humidity are controlled at the open surface and optionally in the vicinity of the membrane penetration cell.

9. Method according to claim 7 or claim 8, wherein as an acceptor phase, water, saline, Ringer-solution, phosphate buffered saline, an aqueous solution of a surfactant or an aqueous solution of a protein, preferably human serum albumin are used.

10. Method according to any of claims 7 to 9, wherein the test is carried out at constant temperature.

## Patentansprüche

1. Vorrichtung zum Testen von kosmetischen oder pharmazeutischen Formulierungen, die zur transdermalen Verwendung bestimmt sind, umfassend ein Konditionierungs- und Zuführungssystem für eine Akzeptorphase (1) und ein System zur Membranpenetration (2), das ein Penetrationselement (C1) aufweist, **dadurch gekennzeichnet, dass** das Penetrationselement (C1) ein Membranpenetrationselement mit offenem Probenraum ist, das einen Zellenkörper umfasst, der aus einem, gegenüber der Akzeptorphase und einer Probenzubereitung inerten Material gebildet ist, wobei in dem Zellenkörper eine Kammer für die Akzeptorphase angeordnet ist und mit einem Einlass und einem Auslass für die Akzeptorphase ausgestattet ist, und auf dem oberen Abschnitt des Zellenkörpers eine Membran angeordnet ist und durch Befestigungsmittel in Position gehalten wird, wobei die Membran zur Bereitstellung der Probenzubereitung auf einer Oberseite der Membran dient und wobei ein Bereich, der die Probenzubereitung enthält, zur Umwelt hin offen ist, wobei der Zellenkörper ein solches Gewicht und eine solche Wärmekapazität aufweist, dass der Fluidstrom bereits im Einlass auf die Temperatur der Zelle gebracht wird und das System zur Membranpenetration keine eigene Temperatursteuerung für die Akzeptorphase umfasst und die Vorrichtung ferner ein Probenentnahme- und ein Analysesystem (3) und ein Steuerungssystem (4) zur Steuerung von ein oder mehreren Umweltfaktoren auf der Membranoberfläche des offenen Probenraums des Membranpenetrationselements umfasst.

2. Vorrichtung nach Anspruch 1, wobei das gegenüber einer Akzeptorphase und einer Probenzubereitung inerte Material Edelstahl ist.

3. System zur Membranpenetration (2) umfassend ein Penetrationselement (C1), das einen offenen Probenbereich aufweist, wobei das Penetrationselement (C1) einen Zellenkörper umfasst, der aus einem gegenüber einer Akzeptorphase und einer Probenzubereitung inerten Material gebildet ist, wobei in dem Zellenkörper eine Kammer für die Akzeptorphase angeordnet ist, und der Zellenkörper mit einem Einlass und einem Auslass für die Akzeptorphase ausgestattet ist, und auf dem oberen Teil des Zellenkörpers eine Membran angeordnet ist und durch Befestigungsmittel in Position gehalten wird, wobei die Membran zur Bereitstellung der Probenzubereitung auf einer Oberfläche der Membran dient, wobei der Bereich, der eine Probenzubereitung enthält, zur Umwelt hin geöffnet ist, wobei der Zellenkörper ein solches Fassungsvermögen und eine solche Wärmekapazität aufweist, dass der Fluidstrom bereits im Einlass auf die Temperatur der Zelle gebracht wird und das System zur Membranpenetration keine eigene Temperatursteuerung für die Akzeptorphase, kein Temperatursteuerungssystem (C2, C3, C4) oder Mittel (L1, L2, A1, A2, A3) umfasst das zur Regulierung und Steuerung einer oder mehrerer Umweltfaktoren auf der Oberfläche der offenen Probenkammer des Membranpenetrationselements geeignet ist.

4. System zur Membranpenetration (2) nach Anspruch 3, **dadurch gekennzeichnet, dass** die einstellbaren und steuerbaren Umweltvariablen des offenen Probenraums des Membranpenetrationselements ausgewählt sind aus Lichtintensität, Lichtwellenlänge, Luftströmungsrate, Lufttemperatur und Luftfeuchtigkeit.

5. System zur Membranpenetration (2) nach Anspruch 3 oder 4, **dadurch gekennzeichnet, dass** das Penetrationselement (C1) mit einem offenen Probenraum einen Wärmeisolator umfasst, der auf der Außenseite des Zellenkörpers bereitgestellt ist.

6. System zur Membranpenetration (2) nach einem der Ansprüche 3 bis 5, wobei das gegenüber einer Akzeptorphase und einer Probenzubereitung inerte Material Edelstahl ist.

7. Verfahren zum Testen von kosmetischen oder therapeutisch medizinischen Zubereitungen, die zur transdermalen Verwendung bestimmt sind, umfassend das Überführen einer Probe auf die Membran, die auf der Oberfläche der Penetrationseinheit gemäß Anspruch 3 angebracht ist, Aussetzen der Probe gegenüber vorausgewählter und steuerbarer Umweltfaktoren unter gleichzeitigem Zuführen eines gesteuerten Fluss an Akzeptorphase, die eine konstante Temperatur aufweist, durch den Zellenkörper und Bestimmen der Konzentration des pharmazeutisch aktiven Bestandteils oder einer für die Testsubstanz charakteristischen Komponente in dem Abfluss.

8. Verfahren nach Anspruch 7, **dadurch gekennzeichnet, dass** ein oder mehrere Umweltfaktoren ausgewählt aus Temperatur, Lichtintensität, Lichtwellenlänge, Luftströmungsrate, Lufttemperatur, Luftfeuchtigkeit auf der offenen Oberfläche und optional in der Nähe der Membranpenetrationseinheit überwacht werden.

9. Verfahren nach Anspruch 7 oder 8, wobei als Akzeptophase Wasser, Kochsalzlösung, Ringerlösung, phosphatgepufferte Kochsalzlösung, eine wässrige Lösung eines Tensids oder eine wässrige Lösung eines Protein, vorzugsweise humanes Serumalbumin, verwendet wird.

10. Verfahren nach einem der Ansprüche 7 bis 9, wobei die Testung unter konstanten Temperaturen erfolgt.

## Revendications

1. Appareil pour le test de formulations cosmétiques ou pharmaceutiques destinées à l'usage transdermique, qui comprend un système de conditionnement et délivrance de phase acceptrice (1) et un système de pénétration membranaire (2) ayant une cellule de pénétration (C1), **caractérisé en ce que** la cellule de pénétration (C1) est une cellule de pénétration membranaire à compartiment d'échantillon ouvert comprenant un corps cellulaire réalisé en un matériau inerte par rapport à une phase acceptrice et une préparation de test, dans lequel une chambre pour la phase acceptrice est située dans le corps cellulaire et est pourvue d'une entrée et sortie de phase acceptrice, et d'une membrane située sur la partie supérieure du corps cellulaire et maintenue en place par des moyens convenant à la fixation, dans lequel la membrane est destinée à localiser la formulation de test sur une surface supérieure de la membrane et dans lequel un compartiment contenant la préparation de test est ouvert vers un environnement, dans lequel le corps cellulaire a un poids et une capacité thermique tels que le courant fluidique est amené à la température de la cellule dès l'entrée et le système de pénétration membranaire ne comprend pas de commande de température séparée pour la phase acceptrice, et l'appareil comprend en outre un échantillonnage et un système analytique (3) et un système de commande (4) pour commander un ou plusieurs facteurs environnementaux au niveau de la surface membranaire du compartiment d'échantillon ouvert de la cellule de pénétration membranaire.

2. Appareil selon la revendication 1, dans lequel le matériau inerte par rapport à une phase acceptrice et une préparation de test est de l'acier inoxydable.

3. Système de pénétration membranaire (2) comprenant une cellule de pénétration (C1) ayant un compartiment d'échantillon ouvert, dans lequel la cellule de pénétration (C1) comprend un corps cellulaire réalisé en un matériau inerte par rapport à une phase acceptrice et une préparation de test, dans lequel une chambre pour la phase acceptrice est située dans le corps cellulaire et le corps cellulaire est pourvu d'une entrée et sortie de phase acceptrice, et d'une membrane située sur la partie supérieure du corps cellulaire et est maintenue en place par des moyens convenant à la fixation, dans lequel la membrane est destinée à localiser la formulation de test sur une surface supérieure de la membrane, dans lequel le compartiment contenant une préparation de test est ouvert vers un environnement, dans lequel le corps cellulaire a un poids et une capacité thermique tels que le courant fluidique est amené à la température de la cellule dès l'entrée et le système de pénétration membranaire ne comprend pas de commande de température séparée pour la phase acceptrice, de système de commande de température (C2, C3, C4) et de moyens (L1, L2, A1, A2, A3) convenant à l'ajustement et la commande d'un ou plusieurs facteurs environnementaux au niveau de la surface du compartiment d'échantillon ouvert de la cellule de pénétration membranaire.

4. Système de pénétration membranaire (2) selon la revendication 3, **caractérisé en ce que** les variables environnementales pouvant être ajustées et commandées au niveau du compartiment d'échantillon ouvert de la cellule de pénétration membranaire sont sélectionnées parmi l'intensité lumineuse, la longueur d'onde lumineuse, le débit d'air, la température de l'air et l'humidité de l'air.

5. Système de pénétration membranaire (2) selon la revendication 3 ou 4, **caractérisé en ce que** la cellule de pénétration (C1) ayant un compartiment d'échantillon ouvert comprend une isolation thermique prévue sur le côté externe du corps cellulaire.

6. Système de pénétration membranaire (2) selon une des revendications 3 à 5, dans lequel le matériau inerte par rapport à une phase acceptrice et une préparation de test est de l'acier inoxydable.

7. Procédé de test de préparations cosmétiques ou médicinales thérapeutiques destinées à l'usage transdermique, qui comprend transférer un échantillon à la membrane reliée à la surface de la cellule de pénétration selon la revendication 3, exposer ledit échantillon à des facteurs environnementaux présélectionnés et commandés avec la délivrance simultanée d'un flux commandé de phase acceptrice à température constante à travers le corps cellulaire et déterminer la concentration de l'ingrédient pharmaceutiquement actif ou d'un constituant caractéristique de l'échantillon de test dans l'effluent.

8. Procédé selon la revendication 7, **caractérisé par le fait qu'**un ou plusieurs facteurs environnementaux sélectionnés parmi la température, l'intensité lumineuse, la longueur d'onde lumineuse, le débit d'air, la température de l'air, l'humidité de l'air sont commandés au niveau de la surface ouverte et en option à proximité de la cellule de pénétration membranaire.

9. Procédé selon la revendication 7 ou la revendication 8, dans lequel de l'eau, de la solution saline, de la solution de Ringer, de la solution saline avec tampon phosphate, une solution aqueuse d'un tensioactif ou une solution aqueuse d'une protéine, de préférence de la sérum albumine humaine, sont utilisées en tant que phase acceptrice.

10. Procédé selon l'une quelconque des revendications 7 à 9, dans lequel le test est effectué à température constante.
